# EUROPÄISCHE PATENTSCHRIFT

(11) **EP 1 084 683 B1**
(45) Veröffentlichungstag und Bekanntmachung des Hinweises auf die Patenterteilung: **04.01.2006**
(21) Anmeldenummer: 00890282.7
(22) Anmeldetag: 15.09.2000
(51) Int. Cl.: A61C 8/00, A61C 3/00, G01L 25/00

(54) **Verfahren und Vorrichtung zur Begrenzung des Drehmomentes zahnärztlicher und chirurgischer Handstücke**
Method and device for limiting the moment of momentum in dental and chirurgical handpieces
Méthode et dispositif pour limiter le moment angulaire des pièces à main dentaires et chirurgicales

(30) Priorität: 17.09.1999 AT 159599
(43) Veröffentlichungstag der Anmeldung: 21.03.2001
(73) Patentinhaber: W & H Dentalwerk Bürmoos GmbH, 5111 Bürmoos (AT)
(72) Erfinder: Sevcik, Roland, 83435 Bad Reichenhall (DE); Fersterer, Johann, 5112 Lamprechtshausen (AT)
(74) Vertreter: Patentanwälte BARGER, PISO & PARTNER

(56) Entgegenhaltungen:
- EP-A- 0 890 344
- WO-A-98/27886
- WO-A-98/44863
- DE-A- 19 845 871
- US-A- 5 898 112

## Beschreibung

Die Erfindung betrifft ein Verfahren, um mittels einer Motorsteuerung zu einer Begrenzung bzw. Eingrenzung des Drehmomentes zahnärztlicher und chirurgischer Handstücke beim Einschrauben von Implantaten bzw. Implantatträgern auf einen vorbestimmten Wert zu kommen, und eine Vorrichtung zur Durchführung des Verfahrens, entsprechend den Oberbegriffen der Ansprüche 1 bzw. 4.

Ein solches Verfahren ist aus der EP 0 890 344 A bekannt. Dort wird davon ausgegangen, dass die Motorsteuerung den Zusammenhang zwischen Drehmoment und Motorstrom prinzipiell kennt und gespeichert hat, und dass die Anpassung an ein spezielles Handstück durch Messung zumindest eines Referenzpunktes der Kurve und anschließendes, angepaßtes Verschieben erfolgt. Die Messung erfolgt mittels eines Drehmomentmessgerätes auf dessen Aufnahmewelle das Handstück direkt aufgesetzt wird.

Aus der WO98/44863 A ist es bekannt, für das Einschrauben eines Implantats in einen Knochen ein Werkzeug für ein Handstück zu gebrauchen, dass an seinem freien Ende eine kugelig-polygonalen Ausbildung aufweist und mit einem passend ausgebildeten Hohlraum der Schraube zusammenwirkt. Es wird gemäß dieser Druckschrift aber die Drehmomemtkurve nicht überprüft sondern es werden (unausgesprochen) die Werksangaben verwendet.

Es ist Aufgabe der Erfindung, ein Verfahren anzugeben, bei dem tatsächlich der Motorstrom gemessen wird, der beim vorbestimmten Drehmoment auftritt und eine Vorrichtung, die es ermöglicht, die Messung durchzuführen, ohne das Handstück zu kontaminieien. Auch dieses Verfahren wird weiter unten näher erläutert.

Dies wird durch die Kennzeichen der Ansprüche 1 bzw. 4 erreicht

Es gibt eine ganze Reihe von chirurgischen Eiuagriffen, insbesondere im Kieferbereich bei der Zahnimplantation, bei denen Implantate (bzw. Implantatträger, auch Verschlußschrauben oder Suprastrukturschrauben, im folgenden der Einfachheit halber generell als Implantat bezeichnet), die mit Gewinden versehen sind, in vorgebohrte Löcher im Knochen eingeschraubt werden sollen. Dazu ist es notwendig, das Drehmoment des elektrisch betriebenen Werkzeuges, mit dem das Einschrauben erfolgt, auf einen vorbestimmten Wert zu begrenzen um ein zu tiefes Einschrauben, eine Beschädigung der Knochenmasse oder ein Ausreissen des meist durch das Implantatgewinde geschnittenen Gewindes zuverlässig zu vermeiden.

So werden beispielsweise sofort nach dem Setzen des Implantates Verschlußschrauben eingeschraubt und es wird (bei Arbeiten im Kieferbereich) das Zahnfleisch über dem Implantat geschlossen und einheilen gelassen. Nach etwa einem halben Jahr oder auch länger wird die Verschlußschraube entfernt und es wird mittels einer Suprastrukturschraube ein Implantataufbau mit dem Implantat verbunden. Diese Suprastrukturschraube muß so fest angezogen werden, dass die Suprastruktur (beispielsweise ein künstlicher Zahn) keinesfalls locker am Implantat sitzt, darf aber beim Anziehen keinesfalls das Implantat aus dem Knochen reissen.

Aufgrund von Erfahrungswerten und Untersuchungen am Patienten wird das maximal anzuwendende Drehmoment bestimmt und es besteht nunmehr das Problem, dieses vorgegebene Drehmoment durch das verwendete Werkzeug, beispielsweise ein Winkelstück, nicht zu überschreiten.

Bei einem bekannten Verfahren verwendet man den vom Produzenten her bekannten und vorgegebenen Zusammenhang zwischen Drehmoment und Motorstrom, um durch permanente Messung des Motorstroms bei Erreichen des vorbestimmten Wertes entweder diesen Strom zu begrenzen oder den Motor stromlos zu schalten und somit auch den Einschraubvorgang zu beenden. Bei diesem Verfahren hat es sich als nachteilig erwiesen, dass durch Alterungsprozesse des Werkzeuges, durch schlechtes oder übermäßiges Schmieren, durch Abnutzung der mechanischen Getriebe oder auch durch Austausch der mechanischen Getriebe bedeutende Abweichungen des tatsächlich aufgebrachten Drehmomentes vom voreingestellten Drehmoment bei gleichem Motorstrom die Folge waren.

Dies führt dazu, dass Implantate entweder zu fest eingeschraubt werden, wobei all die genannten Problemen und Verletzungen auftreten können, oder zu locker, was dazu führt, dass sich nicht nur die Implantatträger, sondern auch das daran montierte Implantat lockern oder gar lösen und eine erneute Behandlung des Patienten, wenn dies überhaupt möglich ist, notwendig wird.

Eine ebenfalls auftretende Gefahr ist das genannte zu lockere Einschrauben derartiger Implantatteile, sei es zufolge unrichtig kalibrierter Handstücke oder wegen des falsch eingeschätzten notwendigen Drehmomentes.

Da, wie oben ausgeführt, erfindungsgemäß die Messung unmittelbar vor dem Einschrauben erfolgt, können sämtliche Toleranzen und Ungenauigkeiten ausgeglichen werden, da ja bei der Messung und der unmittelbar daran anschließenden Behandlung die gleichen Verhältnisse vorliegen. Unter "unmittelbar vorausgehend" bzw. "unmittelbar nachfolgend" wird im Sinne der Erfindung verstanden, dass zwischen der Messung und der Behandlung keine Reinigungs-, Schmier- oder Wartungstätigkeiten oder gar eine Zerlegung des Handstückes vorgenommen wird.

Die erfindungsgemäße Vorrichtung weist eine Vorrichtung zur Aufbringung eines konstanten, einstellbaren Bremsdrehmomentes auf, bevorzugt eine Hysteresebremse, und eine Vorrichtung zur Messung des Motorstromes, sodass bei der Kalibrierung das chirurgische Handstück mit dem voreingestellten Drehmoment belastet wird und der Zusammenhang zwischen dem vorbestimmten Drehmoment und dem Motorstrom eindeutig festgestellt wird.

Derartige Hysteresebremsen sind beispielsweise aus der Textilindustrie, der Papierindustrie, der Folienherstellung, der Messtechnik und verschiedenen anderen Anwendungsgebieten bekannt und industriell erhältlich.

Die Wirkungsweise solcher Hysteresebremsen beruht auf magnetischer Kraftwirkung sich anziehender Pole im Synchronlauf und auf ständiger Ummagnetisierung eines dauermagnetischen Materials im Schlupfbetrieb. Als Hersteller sei auf die MOBAC GmbH in Kiel, Bundesrepublik Deutschland oder die ZF Friedrichshafen AG in Friedrichshafen, Bundesrepublik Deutschland, verwiesen.

Die Erfindung wird im folgenden unter Bezugnahme auf die rein schematische Zeichnung näher erläutert. Diese zeigt eine erfindungsgemäße Messvorrichtung und ein Detail im vergrößerten Maßstab.

Um vor dem Einschrauben eines Implantatteiles die erfindungsgemäß vorgesehene Kalibrierung des Motorstroms durchzuführen, wird ein chirurgisches Handstück 1 mit einem dazu geeigneten Verbindungsstift 2, der mit einem Ende in die Werkzeugaufnahme des Handstückes eingespannt wird, versehen. Beim Kalibriervorgang greift der Verbindungsstift 2 mit seinem unteren Ende, bevorzugt formschlüssig, in eine Aufnahme 3 einer Hysteresebremse 4 ein.

Der Verbindungsstift 2 ist aus sterilisierbaren Material und wird vor jeder Benutzung sterilisiert, um die Werkzeugaufnahme des Handstückes nicht zu kontaminieren. Weiters wird er bevorzugt relativ groß dimensioniert, um jede Berührung zwischen dem sterilisierten Handstück und der nicht sterilisierbaren Aufnahme 3 der Hysteresebremse 4 zu vermeiden.

Die Aufnahme 3 ist, wie aus der Detailzeichnung ersichtlich ist, so ausgestaltet, daß Verkantungen des Verbindungsstiftes 2 gegenüber der Drehachse der Hysteresebremse bis zu etwa 20° problemlos ausgeglichen werden.

Dies wird durch das im wesentlichen kugelig-polygonale Ende 5 des Verbindungsstiftes 2 erreicht, das in eine entsprechend hohlpolygonale Ausnehmung der Aufnahme 3 der Hysteresebremse 4 eingreift und von der Ausnehmung umschlossen wird.

Die Hysteresebremse 4 ist auf den vom Chirurgen vorgegebenen Drehmomentwert eingestellt, der Motor des Handstückes 1 wird in Betrieb genommen und der Motorstrom laufend erhöht, bis das voreingestellte Drehmoment erreicht ist. Während dieser Zeit wird der Motorstrom laufend gemessen und so festgestellt, welcher Motorstrom beim voreingestellten Drehmoment auftritt.

Die Handstückregelung wird sodann entweder händisch oder durch eine Regelautomatik, die dann bevorzugt auch mit der Hysteresebremse und deren Schaltung verbunden ist und deren Werte direkt übernimmt, auf diesen Wert als Maximalwert eingestellt, der Verbindungsstift 2 wird aus dem Handstück entnommen und das einzuschraubende Implantat wird damit verbunden und sodann in den Knochen geschraubt.

Dabei wird die Regelung des Antriebes des Handstückes so vorgenommen, daß das Implantat sich mit konstanter, voreingestellter Geschwindigkeit dreht, wodurch im Laufe des Einschraubvorganges, bei dem ja oft auch erst das Gewinde im Knochen geschnitten wird, das zur Konstanthaltung der Drehgeschwindigkeit notwendige Drehmoment sich laufend erhöht, was die Regelung des Handstückmotors veranlasst, den Motorstrom laufend zu erhöhen.

Diese Erhöhung des Motorstromes wird von einer zweiten Regelstufe überwacht und es wird von dieser zweiten Regelstufe beim Erreichen des unmittelbar zuvor bestimmten Motorstromes der Motor stromlos geschalten und damit der Antrieb ausgeschalten. Es kann sodann durch Lösen der Werkzeug- bzw. Werkstückspannvorrichtung das nunmehr eingeschraubte Implantat vom Handstück getrennt werden und es ist sichergestellt, daß es mit dem vorgegebenen Drehmoment in den Knochen eingeschraubt worden ist.

Da die Regelung des Motorstromes im Stand der Technik an sich bekannt ist, und sich für den Fachmann der Regel- und Steuertechnik keine Probleme bei der Verbindung der Hysteresebremse/Stromstärkemessung mit der Motorsteuerung ergeben, wird darauf hier nicht weiter eingegangen.

In einer vorteilhaften Ausgestaltung der Erfindung ist vorgesehen, daß der tatsächliche Motorstrom, somit das tatsächliche Drehmoment, während des Einschraubvorganges bestimmt und zumindest dessen Maximalwert gespeichert wird. Damit ist es dem Benutzer möglich, wenn er den Einschraubvorgang noch vor dem Erreichen des Ausschaltstromwertes beenden muß, weil er sieht, daß das Implantat die vorgesehene Einschraubtiefe erreicht hat, festzustellen, wie der Drehmomentverlauf bzw. wie groß das maximal aufgetretene Drehmoment war.

Dieses Wissen ermöglicht zumindest eine ex-post Abschätzung der erzielten Haltekraft. Wenn der gesamte Drehmomentverlauf bekannt ist, kann auch abgeschätzt werden, ob es u.U. zum "Abreissen" des im Kiefer geschnittenen Gewindes gekommen ist. Dies wäre z.Bsp. der Fall, wenn anschließend an eine Periode mit relativ hohem Drehmoment dieses wieder abfällt. Durch dieses Wissen können die weiteren notwendigen Dispositionen mit wesentlich größerer Sicherheit als bisher getroffen werden.

Die Erfindung ist nicht auf das beschriebene und dargestellte Ausführungsbeispiel beschränkt, sondern kann verschiedentlich abgewandelt werden. So ist es zur weiteren Erhöhung der Übereinstimmung der Vorgangsweisen beim Kalibrieren und Schrauben möglich, auch beim Kalibrierschritt das Handstück mit der Drehgeschwindigkeit des Einschraubschrittes anzutreiben und die Hysteresebremse kontinuierlich oder schrittweise bis zum vorgegebenen Drehmoment auszusteuern und dabei den Strom zu messen.

Es werden jedenfalls durch die erfindungsgemäßen Maßnahmen die bisher auftretenden Fehler, einschließlich des "worst case" Szenarios, bei dem eine falsche Übersetzung im Winkelhandstück eingesetzt ist, vermieden, ohne daß eine komplizierte (und damit wieder fehleranfällige) oder zeitraubende Prozedur vorzunehmen wäre. Es ist nur der Verbindungsstift einzuspannen, die Kalibriervorrichtung zu aktivieren und nach erfolgter Bestimmung des Maximalwertes des Motorstroms der Verbindungsstift durch das Implantat zu ersetzen, worauf dieses eingeschraubt werden kann.

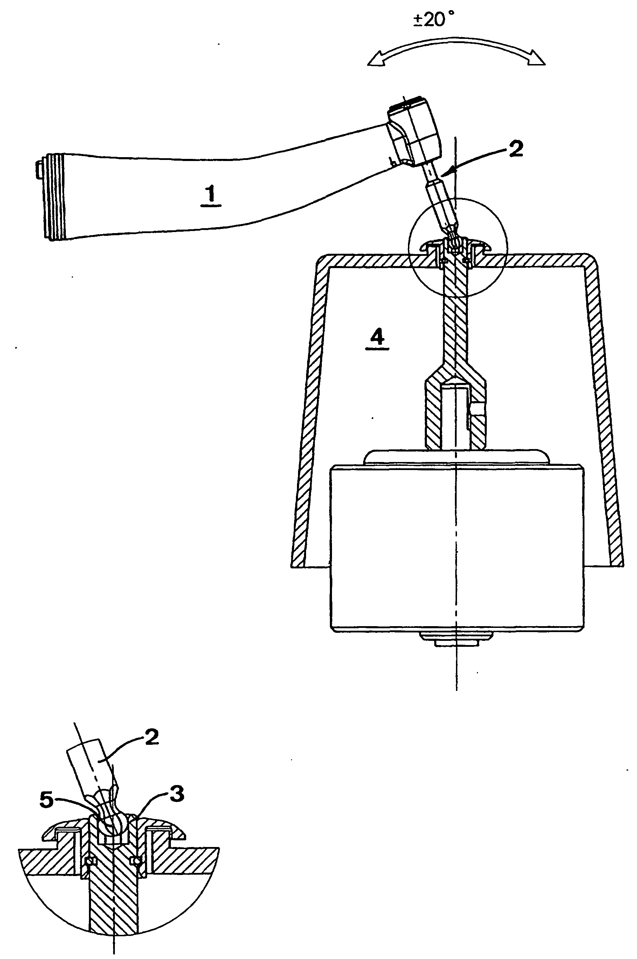

## Patentansprüche

1. Verfahren, um mittels einer Motorsteuerung zu einer Begrenzung bzw. Eingrenzung des Drehmomentes zahnärztlicher und chirurgischer Handstücke beim Einschrauben von Implantaten bzw. Implantatträgern auf einen vorbestimmten Wert zu kommen, **dadurch gekennzeichnet, dass** unmittelbar vor dem Einschrauben eines Implantates bzw. eines Implantatträgers mittels des chirurgischen Handstückes der zur Aufbringung des vorbestimmten Drehmomentes benötigte Motorstrom bestimmt wird, dass dieser bestimmte Wert des Motorstroms in der Motorsteuerung als maximal zulässiger Wert eingegeben wird, und dass durch die Motorsteuerung der Motorstrom beim nachfolgenden Einschraubvorgang auf diesen bestimmten Wert begrenzt wird.

2. Verfahren nach Anspruch 1, **dadurch gekennzeichnet, dass** zwischen der Bestimmung des Motorstromes und dem Einschraubvorgang keine Zerlegung, Reinigung, Schmierung oder andere Wartung des Handstückes vorgenommen wird.

3. Verfahren nach Anspruch 1 oder 2, **dadurch gekennzeichnet, dass** während des Einschraubvorganges der tatsächliche Motorstrom kontinuierlich oder periodisch bestimmt wird, und dass zumindest dessen Maximalwert abrufbar/anzeigbar gespeichert wird.

4. Vorrichtung zur Durchführung des Verfahrens nach Ansprach 1, die eine Drehmomentbremse (4), bevorzugt eine Hysteresebremse, die mit der Werkzeugaufnahme eines Handstückes (1) verbindbar ist, eine Verbindung für das Handstück und eine Motorstrommessvorrichtung für den Handstückmotor aufweist, **dadurch gekennzeichnet, dass** die Verbindung zwischen der Drehmomentbremse (4) und dem Handstück (1) ein Verbindungsstift (2) ist, der einerseits in die Werkzeughalterung des Handstückes (1) einsetzbar und anderseits in eine Aufnahme (3) der Drehmomentbremse (4) lösbar einführbar ist.

5. Vorrichtung nach Anspruch 4, **dadurch gekennzeichnet, dass** der Verbindungsstift (2) an seinem der Aufnahme (3) der Drehmomentbremse (4) zugeordneten Ende eine kugelig-polygonale Ausbildung aufweist.

6. Vorrichtung nach einem der Ansprüche 4 oder 5, **dadurch gekennzeichnet, dass** die Motorstrommeßvorrichtung mit einer Motorsteuerung für das Handstück (1) verbunden ist.

## Claims

1. A method of restricting or limiting to a predetermined value by means of a motor control the torque of hand-operated dental or surgical devices when screwing in implants or implant supports, **characterised in that** immediately before screwing in an implant or an implant support, by means of the hand-operated surgical device, the motor current required to apply the predetermined torque is determined, **in that** this specific value of the motor current is fed into the motor control as a maximum admissible value, and **in that** during the subsequent screwing-in operation the motor current is limited to this specific value by the motor control.

2. A method according to Claim 1, **characterised in that** between the determination of the motor current and the screwing-in operation no dismantling, cleaning, lubrication or other maintenance of the hand-operated device is carried out.

3. A method according to Claim 1 to 2, **characterised in that** during the screwing-in operation the actual motor current is determined continuously or periodically, and **in that** at least its maximum value is stored in a manner enabling it to be retrieved/displayed.

4. An apparatus for carrying out the method according to Claim 1, which has a torque brake, preferably a hysteresis brake, which can be connected to the tool holder of a hand-operated device (1), has a connection for the hand-operated device and a motor-current measuring device for the motor of the hand-operated device, **characterised in that** the connection between the torque brake (4) and the hand-operated device (1) is a connecting pin (2) which, at one end, can be inserted into the tool holder of the hand-operated device (1) and, at the other end, can be introduced into a socket (3) of the torque brake (4).

5. An apparatus according to Claim 4, **characterised in that**, at its end associated with the socket (3) of the torque brake (4), the connecting pin (2) is of spherical-polygonal shape.

6. An apparatus according to Claim 4 or 5, **characterised in that** the motor-current measuring device is connected to a motor control for the hand-operated device (1).

## Revendications

1. Procédé pour exécuter, à l'aide d'une unité de commande de moteur, une limitation ou réduction du couple de pièces à main de dentisterie et de chirurgie lors du vissage d'implants ou de supports d'implants, à une valeur prédéterminée, **caractérisé en ce que** directement avant le vissage d'un implant ou d'un support d'implant à l'aide de la pièce à main de chirurgie, le courant du moteur, nécessaire pour appliquer le couple prédéterminé, est déterminé, que cette valeur déterminée du courant du moteur est introduite en tant que valeur maximale admissible dans l'unité de commande du moteur et que le courant du moteur est limité par l'unité de commande du moteur à cette valeur déterminée, lors de l'opération de vissage suivante.

2. Procédé selon la revendication 1, **caractérisé en ce qu'**aucun démontage, aucun nettoyage, aucune lubrification ni aucun autre entretien de la pièce à main ne sont exécutés entre la détermination du courant du moteur et le processus de vissage.

3. Procédé selon la revendication 1 ou 2, **caractérisé en ce que** pendant l'opération de vissage, le courant effectif du moteur est déterminé continûment ou périodiquement et qu'au moins sa valeur maximale est mémorisée de manière à pouvoir être appelée/affichée.

4. Dispositif pour la mise en oeuvre du procédé selon la revendication 1, qui comporte un frein de couple (4), de préférence un frein à hystérésis, qui peut être relié au logement de l'outil d'une pièce à main (1), une liaison pour la pièce à main et un dispositif de mesure du courant du moteur pour le moteur de la pièce à main, **caractérisé en ce que** la liaison entre le frein de couple (4) de la pièce à main (1) est une tige de liaison (2), qui d'une part peut être insérée dans le dispositif de retenue de l'outil de la pièce à main (1) et d'autre part peut être introduite de façon amovible dans un logement (3) du frein de couple (4).

5. Dispositif selon la revendication 4, **caractérisé en ce que** la tige de liaison (2) possède une forme de réalisation sphérique-polygonale sur son extrémité tournée vers le logement (3) du frein de couple (4).

6. Dispositif selon l'une des revendications 4 ou 5, **caractérisé en ce que** le dispositif de mesure du courant du moteur est relié à une unité de commande du moteur pour la pièce à main (1).
